Europäisches Patentamt

**European Patent Office**

**Office européen des brevets**

(19)

(11) Publication number: **0 009 977**
**B1**

(12)                    **EUROPEAN PATENT SPECIFICATION**

(45) Date of publication of patent specification: **16.11.83**    (51) Int. Cl.³: **A 61 L  15/00**

(21) Application number: **79302110.6**

(22) Date of filing: **04.10.79**

(54)  **A water-swellable particulate absorbent material and method for producing this material.**

| | |
|---|---|
| (30) Priority: **05.10.78 GB 3937678** | (73) Proprietor: **UNILEVER PLC**<br>**Unilever House Blackfriars  P O Box 68**<br>**London EC4P 4BQ (GB)** |
| (43) Date of publication of application:<br>**16.04.80 Bulletin 80/8** | (84) **GB**<br>(73) Proprietor: **UNILEVER NV**<br>**Burgemeester s'Jacobplein 1 P.O. Box 760**<br>**NL-3000 DK  Rotterdam (NL)** |
| (45) Publication of the grant of the patent:<br>**16.11.83 Bulletin 83/46** | (84) **BE CH DE FR IT LU NL SE AT** |
| (84) Designated Contracting States:<br>**AT BE CH DE FR GB IT LU NL SE** | (72) Inventor: **Bosley, John Anthony**<br>**10 Philip Way**<br>**Higham Ferrers Northamptonshire (GB)**<br>Inventor: **Farmer, Vicki**<br>**12 Clovelly Way**<br>**Bedford (GB)**<br>Inventor: **Mckinnon, Alan Andrew**<br>**13 St. Crispin Way**<br>**Raunds Northamptonshire (GB)** |
| (56) References cited:<br>**FR - A - 1 134 126**<br>**FR - A - 1 548 038**<br>**FR - A - 2 000 595**<br>**FR - A - 2 066 324**<br>**FR - A - 2 187 947**<br>**FR - A - 2 354 184**<br>**GB - A - 1 233 239**<br>**US - A - 2 602 042**<br>**US - A - 3 371 666**<br>**US - A - 3 847 636**<br>**US - A - 4 055 184** | (74) Representative: **Doucy, Robert Henry et al,**<br>**Unilever PLC Patents Division P.O. Box 68**<br>**Unilever House**<br>**London EC4P 4BQ (GB)** |

Courier Press, Leamington Spa, England.

## A water-swellable particulate absorbent material
## and method for producing this material

This invention relates to water-swellable particulate absorbent materials, more especially absorbent materials suitable for use in absorbent disposable products such as sanitary towels or napkins and tampons and absorbent products for surgical or medical use. The invention also relates to methods for producing such materials.

In recent years there has been much interest in the synthesis of absorbent polymers having a high capacity for absorbing water and body fluids. A number of different polymers have been developed some being wholly synthetic in nature and some partially synthetic.

Among the partially synthetic absorbent polymers are those based on starch. These include the starch-acrylo-nitrile graft copolymers described in US Patent Specifications Nos. 3,997,484 (the USA as represented by the Secretary of Agriculture) and 3,661,815 (Grain Processing Corporation), and the cross-linked gelatinised starch derivatives described in German Patent Application No. 2702781. Other examples of partially synthetic absorbent polymers are the carboxymethylated cellulose described in US Patent Specification No. 3,589,364 (Buckeye Cellulose Corporation), and the carboxymethylcellulose cross-linked by acid inter-esterification as described by Podlas, T. J., in INDA Tech. Symp. 1976, 2—3 March, pp. 25—39.

Wholly synthetic absorbents include polyacrylates crosslinked with a polyamide/epichlorhydrin material as described in US Patent Specification No. 4,076,673 (Dow Chemical Company) and the potassium salts of polyacrylic acid cross-linked by aluminium ions as described in US Patent Specification No. 4,090,013 (National Starch and Chemical Corporation).

The above materials are substantially water-insoluble and absorb many times their weight of water, urine or other aqueous body exudates. They retain a particulate character as they imbibe surrounding liquid and swell. In most cases, even with water, the swollen particles are gelatinous and tacky and form clusters. An exception is the cross-linked starch derivative described in German Patent Application No. 2702781, the particles of which on absorbing water are substantially non-tacky and dry to the touch. However, even this starch derivative, as well as the other absorbents previously referred to, has poor dispersibility in blood, so that when blood is brought into contact with a mass of the absorbent significant penetration into the bed and hence absorption is extremely slow. The particles have therefore what may be described as poor "blood wet-out" so that on exposure to blood the particulate material at the inside of a mass remains unreached by the blood. Although the mechanism is not understood at the present time, it appears that the blood on initial contact with the particles forms some sort of mass which acts as a barrier to the passage of the blood through the surface layers of the particles.

By the process described in US—A—2 602 042 methyl cellulose is obtained in a condition to rapidly absorb various liquids. The process involves treatment of the methyl cellulose with certain solutions, such as fractional petroleum distillates, which are subsequently completely removed. FR—A—2 354 184 describes the production of aerated films, the aeration being said to enhance the rate at which the films absorb water. The application of a lubricant coating to one end of a tampon is disclosed in FR—A—1 134 126. In the lubricant film may be dispersed a surface-active agent which is said to increase the speed of dispersion of the film of lubricant into the body fluids. A sanitary napkin is described in FR—A—2 000 595 which comprises means for providing a rapid transfer of viscous mentrual fluid exudate from the surface of the napkin to the underlying absorbent core. This result is achieved by locating a web of non-woven polypropylene fibres between the absorbent core and the surface of the napkin, which fibre web may be treated with a non-ionic wetting agent to improve the rate of fluid transfer from the surface of the napkin to the absorbent core.

None of the prior specifications is concerned with the object of Applicant's invention which is to provide a water-swellable particulate absorbent material having improved blood dispersibility, or "blood wet-out". It is also an object to provide a method for producing such an improved absorbent material.

According to the invention there is provided a substantially water-insoluble water-swellable wholly or partially synthetic polymeric absorbent material having a water-retention value of at least 2 g/g in the form of particles surface-treated to enhance its dispersibility in blood, characterised in that the absorbent particles are treated with a hydrocarbon compound which is a non-volatile aliphatic hydrocarbon, fatty alcohol, fatty acid or fatty acid ester having a melting point below 35°C, or is a $C_{12}$—$C_{18}$ alkyl trimethylammonium halide, the amount of the hydrocarbon compound being from 1 to 30% by weight of the untreated absorbent material and such as to permit capillary flow of blood through a mass of the absorbent material.

The aliphatic hydrocarbon, fatty alcohol, fatty acid or fatty acid ester preferably has a melting point below 30°C, and more preferably is liquid at room temperature. Of these, the most preferred compounds are those which have melting points below 20°C.

By non-volatile we mean that the aliphatic hydrocarbon, fatty alcohol, fatty acid or fatty acid ester has a boiling point of at least 150°C, preferably at least 200°C. Treatment with the non-volatile compounds gives a longer lasting benefit than would be given by volatile compounds.

Examples of suitable aliphatic hydrocarbons are n-dodecane, n-hexadecane, n-heptadecane and n-octadecane. Examples of fatty alcohols are nonan-1-ol, decan-1-ol, undecan-1-ol and do-decan-1-ol. Examples of suitable fatty acids which may be used are n-hexanoic acid, n-octanoic acid, n-nonanoic acid, 9-octadecenoic acid, 9,12-octadecadienoic acid and 9,12,15-octadecatrienoic acid; these fatty acids are substantially nonionic in character, exhibiting very low dissociation constants. Examples of suitable fatty acid esters are the glycerides constituting the edible oils soybean oil, cottonseed oil, corn oil, peanut oil, safflower oil, sunflower oil, olive oil, rapeseed oil, coconut oil and palm kernel oil. Other examples of suitable fatty acid esters are the fatty acid esters of sorbitol and poly-(ethylene oxide) condensates thereof such as sorbitan mono-oleate (available commercially under the trade name SPAN 80) and polyoxyethylene (20) sorbitan monostearate (available commercially under the trade name TWEEN 60); the words SPAN and TWEEN and registered trade marks. However, esters of other polyhydric alcohols may be used. The ester is desirably substantially odourless.

Those aliphatic hydrocarbons, fatty alcohols, fatty acids and fatty acid esters which are solid at room temperature (say 22°C) exert their beneficial effect under conditions at which they are liquid and therefore obsorbents treated with such compounds are required to be used under conditions where the temperature will exceed their melting point, for example in a menstrual tampon.

The $C_{12}$—$C_{18}$ alkyl trimethylammonium halide may, for example, be cetyl trimethylammonium bromide or chloride.

The hydrocarbon compound used for the treatment of the absorbent material should of course be inert and physiologically acceptable.

The water-swellable absorbent materials which may be treated with the hydrocarbon compound are exemplified by the partially synthetic and wholly synthetic absorbent polymers described above. The water-retention values of such polymers are at least 2 g/g and are usually much higher. The particulate absorbent material is in the form of a powder, granules or flakes.

The treatment of the absorbent with a liquid hydrocarbon compound may be effected simply by directly mixing the particulate absorbent with the hydrocarbon compound. When a hydrocarbon compound is used which is solid at normal temperature it is necessary to first melt the hydrocarbon compound and then to thoroughly mix the particulate absorbent with the molten compound. Mixtures of solid and liquid hydrocarbon compounds would also require to be heated to melt the solid component.

The treatment of the absorbent with the hydrocarbon compound may be effected by first dissolving the hydrocarbon compound in a suitable volatile solvent and after thoroughly mixing the absorbent with this solution, heating the mixture to drive off the solvent. Solvents which may be used are the lower aliphatic alcohols, preferably ethanol or isopropanol, although any other solvent could be used which is sufficiently volatile to permit its ready removal after admixture with the absorbent material. It may be possible to produce the surface-treated absorbent by adding the hydrocarbon compound in solution in a solvent during the process of manufacturing the absorbent. For example, in the case of the absorbent described in German Patent Application No. 2702781, the starch derivative in its acid form is conveniently mixed with a solution of the hydrocarbon compound in a volatile solvent and an alkali, e.g. sodium carbonate or ammonium hydroxide, and the mixture heated to obtain as a dry powder the treated starch derivative in the salt form.

The amount of the hydrocarbon compound that is used for the treatment of the absorbent depends on a number of factors. The amount which is sufficient to effect the enhancement of the dispersibility of the absorbent in blood (i.e. the "blood wet-out" of the absorbent) may vary with the chemical type of absorbent and its physical form, e.g. particle size, and the hydrocarbon compound used in the treatment, as well as on the method of treatment. It should be noted, however, that excessive amounts of the hydrocarbon compound inhibit blood dispersibility by preventing capillary flow of blood through a mass of the particulate absorbent. It is required that the blood should be able to percolate through the capillaries or spaces between the particles and therefore these should not be blocked by the hydrocarbon compound. If this occurs the blood would be forced to travel through the mass by diffusion alone: this is a relatively slow process and corresponds to very poor blood wet-out properties. Consequently, the amount of the hydrocarbon compound used for the treatment of the absorbent should not be such as to result in, for example, a mixture having a paste-like or ointment-like consistency such as is produced by the composition described in Example 6 of British Patent Specification No. 1,454,055 (Pharmacia AB).

Employing the gelatinised starch derivatives described in German Patent Application No. 2702781 good results have been obtained using as little as 1—5% by weight of a liquid aliphatic hydrocarbon or solid $C_{12}$—$C_{18}$ alkyl trimethylammonium halide based on the weight of absorbent. The edible oils are generally required to be used in greater amounts than the liquid aliphatic hydrocarbons.

By treatment of an absorbent with a hydrocarbon compound in accordance with the invention one can also improve the feel to the touch of a mass of absorbent swollen with blood. Many absorbent polymers become very sticky to the touch when blood is added to a mass of the particles. We have found that treatment with the hydrocarbon compound reduces this stickiness.

The treatments described do not markedly affect the blood retention value of an absorbent.

The absorbent material of the invention may be incorporated in absorbent articles. The absorbent article may comprise a fibrous carrier or support for the absorbent material, such as a woven or unwoven material such as cotton cloth, rayon, wool, surgical gauze or paper as well as cellulosic fluff,

3

# 0 009 977

on or within which the absorbent material is supported. The absorbent material may be spread on the carrier or it may be mixed with loose fibres to make a composite fluff or wadding which can be enclosed between cover sheets of paper or cloth. The article may also be in the form of a laminate. In a particular form, the carrier comprises two sheets between which the absorbent material is sandwiched. The absorbent materials of the invention are particularly suitable for use in sanitary towels, napkins or tampons. The production of an absorbent article utilising a particulate absorbent material is described for example in German Patent Application No. 2702781.

The invention will now be illustrated by the following Examples. Percentages are by weight.

References herein to the "dry-off" of an absorbent refer to feel of the polymer to the touch after a mass of particles of the absorbent has absorbed blood. An absorbent with poor dry-off would be sticky to the touch whereas one with a good dry-off would feel substantially dry and non-sticky. Water and urine retention values are given to the nearest quarter of a unit. They were determined by the procedure described in German Patent Application No. 2702781.

### Examples 1 to 9

Potato starch (1,000 g) was slurried in water (950 ml) containing epichlorhydrin (8.4 ml; 1.0% epichlorhydrin by weight of starch). Sodium hydroxide (5 g) in water (50 ml) was added with stirring and the mixture was applied to a heated roller via a feeder roller to form a layer on the surface of the roller of about 0.5 mm thickness. The roller itself was heated using steam at 3.77 bars (140°C). The cross-linked starch derivative was removed from the roller as a flake material to yield 914 g of product. The soluble content of the product was found to be 25.0 mg/g and the product was found to have a bed volume of 13.5 ml/g. Since about half of the epichlorhydrin was lost by evaporation from the heated roller the degree of substitution of the cross-linking groups was about 0.01.

Sodium hydroxide (34 g) in water (66 ml) followed by monochloroacetic acid (39 g) in water (11 ml) was slowly added with stirring to the cross-linked potato starch (100 g) as prepared above. The mixture was aged overnight in a polythene bag. The theoretical degree of substitution was 0.67.

The moist carboxymethyl derivative was dispersed in 10 times its weight of 1N hydrochloric acid and soaked for 15 minutes and then filtered. The gel cake was repeatedly dispersed in water and filtered until the filtrate was substantially free of chloride ions. Ammonium hydroxide, specific gravity 0.910 (70 ml) was mixed with the water-swollen washed cake before drying in a forced air oven (70°C) and milling (2 mm screen). The milled product had a water retention value of 20.00 g/g, a urine retention value of 10.25 g/g, a solubility of 0.3% and a bed volume of 51 ml/g.

Various liquid hydrocarbon and hydrocarbon derivatives were used to treat the control material prepared as described above by thoroughly mixing them with the absorbent. The percentages of the hydrocarbons and hydrocarbon derivatives added which are given in Table I are based on the weight of the control material. In each case the treated absorbent was of particulate form.

TABLE I

| Example | Additive | % Added |
|---|---|---|
| 1 | n-dodecane | 1 |
| 2 | n-hexadecane | 1 |
| 3 | decan-1-ol | 5 |
| 4 | n-nonanoic acid | 10 |
| 5 | Olive oil | 5 |
| 6 | Soybean oil | 5 |
| 7 | Peanut oil | 5 |
| 8 | Sorbitan mono-oleate (SPAN 80) | 5 |
| 9 | Polyoxyethylene (20) sorbitan monostearate (TWEEN 60) | 5 |

The products of Examples 1 to 9 showed a marked improvement in blood wet-out and dry-off compared to the control material to which no addition had been made.

The ability of an absorbent to be wet-out by blood was assessed by placing 1—2 g of the absorbent to be treated on a watch glass and adding to it 1—3 ml of blood. In the cases where the

4

absorbent had been treated with a hydrocarbon or hydrocarbon derivative the blood rapidly penetrated the mass of particles whereas in the case of the control material the blood only very slowly, if at all, penetrated the mass to reach the particles in the interior of the mass. All blood wet-out testing was carried out at room temperature (about 22°C).

### Examples 10 to 12

The hydrocarbon n-dodecane was added directly, with thorough mixing, to the absorbent materials as described in Table II, each of which has a water retention value greater than 2 g/g. In each case the blood wet-out and dry-off of the absorbent was improved compared to the respective unmodified absorbent.

#### TABLE II

| Example | Absorbent | % n-dodecane |
|---|---|---|
| 10 | Absorbent A | 10 |
| 11 | Absorbent B | 10 |
| 12 | Absorbent C | 2 |

Absorbent A was the potassium salt of a polyacrylic acid cross-linked by aluminium ions available commercially from National Starch Corporation under the trade name Permasorb and generally described in US Patent Specification No. 4,090,013.

Absorbent B was a carboxymethylated cellulose cross-linked by intermolecular esterification and available from Hercules Corporation under the trade name SPX 1154 and generally described by Podlas, T. J., in INDA Tech. Symp. 1976, 2—3 March, pp. 25—39. It is a powder obtained by comminuting a form of the absorbent.

Absorbent C was a starch-polyacylonitrile graft copolymer prepared in accordance with the process described in US Patent No. 3,981,100.

### Examples 13 to 16

Cetyl trimethylammonium bromide (CTAB), dissolved in a small volume of absolute ethanol, was thoroughly mixed with the absorbent materials indicated in Table III, each of which had a water retention value greater than 2 g/g. The mixtures were placed in a forced air oven at 60°C to remove the solvent. The amount of cetyl trimethylammonium bromide given in Table III is based on the weight of the control material. In each case the blood wet-out and dry-off of the treated material was improved compared to the control material to which no addition had been made.

#### TABLE III

| Example | Absorbent | % CTAB |
|---|---|---|
| 13 | of Example 1 | 5 |
| 14 | Absorbent A | 20 |
| 15 | Absorbent D | 20 |
| 16 | Absorbent E | 30 |

Absorbent D was a hydrolysed starch-polyacrylonitrile graft copolymer available from the Grain Processing Corporation under the trade name Polymer 35-A-100 and generally described in US Patent No. 3,661,815.

Absorbent E was a hydrolysed starch-polyacrylonitrile graft copolymer available from General Mills Inc., under the trade name SCP-502S and generally described in US Patent No. 3,997,484.

In co-pending application No. 78300507.7 (Publication No. 0001706) there is described another means of enhancing the dispersibility in blood of a water-swellable particulate absorbent material, wherein the absorbent material is surface-treated with one or more polyethers containing oxyethylene and/or oxypropylene groups.

### Claims

1. A substantially water-insoluble water-swellable wholly or partially synthetic polymeric absorbent material having a water-retention value of a least 2 g/g in the form of particles surface-treated to enhance its dispersibility in blood, characterised in that the absorbent particles are treated with a hydrocarbon compound which is a non-volatile aliphatic hydrocarbon, fatty alcohol, fatty acid or fatty acid ester having a melting point below 35°C, or is a $C_{12}$—$C_{18}$ alkyl trimethylammonium halide, the amount of the hydrocarbon compound being from 1 to 30% by weight of the untreated absorbent material and such as to permit capillary flow of blood through a mass of the absorbent material.

2. An absorbent material as claimed in Claim 1, characterised in that the aliphatic hydrocarbon, fatty alcohol, fatty acid or fatty acid ester is a liquid.

3. A method of producing an absorbent material as claimed in Claim 1 having enhanced dispersibility in blood, characterised in that the absorbent particles are contacted with one or more of the hydrocarbon compounds as defined in Claim 1 and in an amount as defined in Claim 1 to form a coating of hydrocarbon compound on the surface of the particles.

4. A method as claimed in Claim 3, characterised by intimately mixing the absorbent particles with one or more liquid or liquefied hydrocarbon compounds as defined in Claim 1.

5. A method as claimed in Claim 3, characterised by intimately mixing the absorbent particles with a solution in a volatile solvent of one or more hydrocarbon compounds as defined in Claim 1 and then removing the solvent.

**Revendications**

1. Un matériau absorbant polymérique entièrement ou partiellement synthétique substantiellement insoluble et gonflant dans l'eau ayant une valeur de rétention d'eau d'au moins 2 g/g sous forme de particules à surface traitée pour augmenter son aptitude à la dispersion dans la sang, caractérisé en ce que les particules absorbantes sont traitées avec un composé hydrocarboné qui est un hydrocarbure aliphatique, un alcool gras, un acide gras ou un ester d'acide gras non volatile ayant un point de fusion en dessous de 35°C, ou est un halogénure de $C_{12}$—$C_{18}$ alkyl triméthylammonium, la quantité du composé hydrocarboné étant de 1 à 30% en poids du matériau absorbant non traité et telle qu'elle permette un flux capillaire de sang à travers une masse du matériau absorbant.

2. Un matériau absorbant selon la revendication 1, caractérisé en ce que l'hydrocarbure aliphatique, l'alcool gras, l'acide gras ou l'ester d'acide gras est un liquide.

3. Un procédé d'obtention d'un matériau absorbant selon la revendication 1 ayant une aptitude accrue à la dispersion dans le sang, caractérisé en ce que les particules absorbantes sont mises en contact avec un ou plusieurs des composés hydrocarbonés comme définis dans la revendication 1 et dans une quantité comme définie dans la revendication 1 pour former un revêtement de composé hydrocarboné sur la surface des particules.

4. Un procédé selon la revendication 3, caractérisé par un mélange intime des particules absorbantes avec un ou plusieurs composés hydrocarboné liquides ou liquifiés comme définis dans la revendication 1.

5. Un procédé selon la revendication 3, caractérisé par un mélange intime des particules absorbantes avec une solution dans un solvant volatile d'un ou plusieurs composés hydrocarboné comme définis dans la revendication 1 et ensuite par un enlèvement du solvant.

**Patentansprüche**

1. Praktisch wasserunlösliches, mit Wasser quellbares, voll- oder teilsynthetisches, polymeres, absorbierendes Material mit einem Wasser-Retentionswert von wenigstens 2 g/g in Form von oberflächenbehandelten Teilchen zur Erhöhung seiner Dispergierbarkeit in Blut, dadurch gekennzeichnet, daß die absorbierenden Teilchen mit einer Kohlenwasserstoffverbindung behandelt werden, die ein nicht-flüchtiger aliphatischer Kohlenwasserstoff, Fettalkohol, eine Fettsäure oder ein Fettsäureester mit einem Schmelzpunkt unter 35°C oder ein $C_{12}$—$C_{18}$-Alkyltrimethylammoniumhalogenid ist, wobei die Menge der Kohlenwasserstoffverbindung 1 bis 30 Gew.-% des unbehandelten absorbierenden Materials und so ist, daß Kapillarfluß von Blut durch eine Masse des absorbierenden Materials ermöglicht wird.

2. Absorbierendes Material nach Anspruch 1, dadurch gekennzeichnet, daß der aliphatische Kohlenwasserstoff, Fettalkohol, die Fettsäure oder der Fettsäureester eine Flüssigkeit ist.

3. Verfahren zum Herstellen eines absorbierenden Materials, wie in Anspruch 1 beansprucht, mit erhöhter Dispergierbarkeit in Blut, dadurch gekennzeichnet, daß die absorbierenden Teilchen mit einem oder mehreren der Kohlenwasserstoffverbindungen, wie in Anspruch 1 definiert, und in einer Menge, wie in Anspruch 1 definiert, zur Bildung eines Überzugs aus Kohlenwasserstoffverbindung auf der Oberfläche der Teilchen zusammengebracht werden.

4. Verfahren nach Anspruch 3, gekennzeichnet durch inniges Mischen der absorbierenden Teil-

6

chen mit einem oder mehreren flüssigen oder verflüssigten Kohlenwasserstoffverbindungen, wie in Anspruch 1 definiert.

5. Verfahren nach Anspruch 3, gekennzeichnet durch inniges Mischen der absorbierenden Teilchen mit einer Lösung einer oder mehrerer Kohlenwasserstoffverbindungen, wie in Anspruch 1 definiert, in einem flüchtigen Lösungsmittel und dann Entfernen des Lösungsmittels.